# EUROPEAN PATENT APPLICATION

(11) **EP 4 516 920 A1**
(43) Date of publication of application: **05.03.2025**
(21) Application number: 23790862.9
(22) Date of filing: 17.02.2023
(51) Int. Cl.: C12P 19/34

(54) **CAPPED COMPOSITION AND PREPARATION METHOD THEREFOR, AND IN-VITRO TRANSCRIPTION REACTION SYSTEM**

(30) Priority: 23.04.2022 CN 202210430616
(71) Applicant: Jiangsu Synthgene Biotechnology Co., Ltd., Nanjing, Jiangsu 210000 (CN)
(72) Inventor: HUANG, Lei, Nanjing, Jiangsu 210000 (CN); TONG, Kun, Nanjing, Jiangsu 210000 (CN); XIAO, Xiao, Nanjing, Jiangsu 210000 (CN)
(74) Representative: Mewburn Ellis LLP
(86) International application number: PCT/CN2023/076711
(87) International publication number: WO 2023/202199

(57) **Abstract**

Provided are a capped composition and a preparation method therefor, and an in-vitro transcription reaction system. The composition substantially comprises a salt solution compounded by a capped analogue and TRIS according to a molar ratio of 1:(3-7). Due to the high biocompatibility of TRIS, when some specific mRNA sequences are transcribed, TRIS has more excellent biocompatibility with mRNA, so that the in-vitro transcription efficiency of mRNA can be increased, and the capping efficiency of mRNA can be significantly increased.

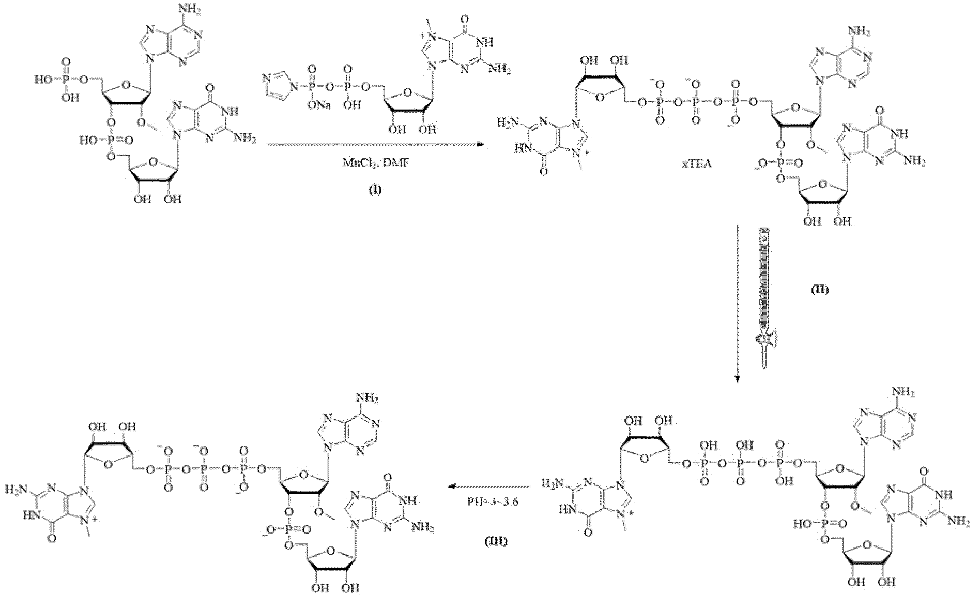

## Description

### TECHNICAL FIELD

The present disclosure relates to the field of chemical and biological engineering, and particularly to a capped composition and methods of making thereof, and an *in vitro* transcription reaction system.

### BACKGROUND

The synthesis of mRNA by *in vitro* transcription has become an important tool for the introduction of foreign genes to express proteins, and is widely applied to the treatment and prevention of diseases. The process of chemically capping mRNA is a critical step in the synthesis of mRNA by *in vitro* transcription.

However, the systems used for mRNA capping are complex. For example, capping enzymes have poor recognition efficiency for some specific mRNA sequences, leading to low capping efficiency. Some mRNA sequences have a lot of secondary structures, thereby causing significant steric hindrance during the capping process, and ultimately resulting in low capping efficiency.

Therefore, there is an urgent need in the art to develop a new class of capping analog combinations to achieve a higher capping efficiency for mRNA synthesized via *in vitro* transcription.

### DETAILED DESCRIPTION

A first objective of the present disclosure is to provide a capped composition with TRIS salt, which can significantly improve the capping efficiency.

A second objective of the present disclosure is to provide a method of making the capped composition, which is an efficient way to prepare a stable capped composition.

A third objective of the present disclosure is to provide an *in vitro* transcription reaction system comprising the capped composition described herein, with which to provide high capping efficiency and high yield for the mRNA synthesized; and to improve the cellular protein expression levels when compared to the controls using other types of capped compositions.

In a first aspect, the present disclosure provides a capped composition, adopting the following technical solutions:

A capped composition, wherein the capped composition substantially comprises a salt solution compounded by a capped analogue and TRIS according to a molar ratio of 1:(3-7).

The present disclosure applies a TRIS salt to a biological enzymatic catalysis system. Specifically, the capping analog and TRIS are compounded by a molar ratio of 1:(3-7) to form a salt solution, thereby obtaining a capped composition. Due to the high biocompatibility of TRIS, when transcribing some specific mRNA sequences are transcribed, TRIS has better biocompatibility with mRNA, so that the yield of mRNA transcription can be increased, and the capping efficiency of mRNA can be significantly increased.

Preferably, the capping analog has a formula: 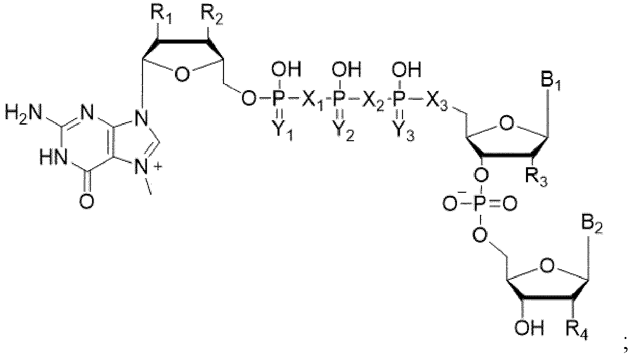
the TRIS has a formula of

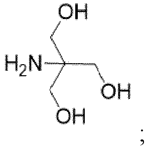

wherein, each of B₁ and B₂ is independently a natural or modified base;
R₁ is H, OH, alkyl, O-alkyl, or halogen;
R₂ is H, OH, alkyl, O-alkyl, or halogen;
R₃ is hydrogen, OH, substituted or unsubstituted O-alkyl, substituted or unsubstituted S-alkyl, substituted or unsubstituted NH-alkyl, substituted or unsubstituted N-dihydrocarbyl, substituted or unsubstituted O-aryl, substituted or unsubstituted S-aryl, substituted or unsubstituted NH-aryl, substituted or unsubstituted O-arylalkyl, substituted or unsubstituted S-arylalkyl, or substituted or unsubstituted NH-arylalkyl;
R₄ is H, OH, substituted or unsubstituted O-alkyl, substituted or unsubstituted S-alkyl, substituted or unsubstituted NH-alkyl, substituted or unsubstituted N-dihydrocarbyl, substituted or unsubstituted O-aryl, substituted or unsubstituted S-aryl, substituted or unsubstituted NH-aryl, substituted or unsubstituted O-arylalkyl, substituted or unsubstituted S-arylalkyl, or substituted or unsubstituted NH-arylalkyl;
each of X₁, X₂, and X₃ is independently O, CH₂, or NH; and
each of Y₁, Y₂, and Y₃ is independently O, S, Se, or BH₃.

Preferably, the capping efficiency when using the capped composition is 98% or more.

By adopting the technical solutions described herein, the capping efficiency when using the capped composition according to the formula and the preparation method of the present disclosure can be 98% or more. Experiments show that the salt solution formed by compounding TRIS with the capping analog has significantly improved the capping efficiency when compared to a sodium salt capped composition or an ammonium salt capped composition. This is an unexpected result.

Preferably, the capped composition increases the yield of mRNA by 45% or more in *in vitro* transcription, as compared to a sodium salt capped composition or an ammonium salt capped composition.

By adopting the technical solutions described herein, using the salt solution formed by compounding of a capping analog with TRIS increases the yield of mRNA significantly, by 45% or more, in *in vitro* transcription when compared to a sodium salt capped composition or an ammonium salt capped composition. In some embodiments, the yield of synthetic mRNA when using the salt solution formed by compounding the capped analog with TRIS reaches 72 µg, whereas the yields of synthetic mRNA when using a sodium salt capped composition or an ammonium salt capped composition are less than 50 µg.

Preferably, the capped composition increases the intracellular protein translation efficiency of mRNA by 1.25 times or more after *in vitro* transcription, as compared to a sodium salt capped composition or an ammonium salt capped composition.

After *in vitro* transcription is carried out by using the capped composition of the present disclosure, the intracellular mRNA protein translation efficiency is increased by 1.25 times or more when compared to that of a sodium salt capped composition or an ammonium salt capped composition. The intracellular protein expression is better, and the transcription and translation effect are better.

In a second aspect, the present disclosure provides a method of making a capped composition, adopting the following technical solutions:

Provided is a method of making a capped composition, comprising the following steps:
dissolving an m7GDP imidazole salt in a DMF solution comprising MnCl₂, adding the DMF solution of m7GDP imidazole salt to a DMF solution of 5'-phosphorylated dinucleotide, stirring the mixture at room temperature, and concentrating to obtain a triethylamine salt of the capped analog;
converting the triethylamine salt of the capped analog to a free acid of the capped analog by a cation exchange resin; and
adding a TRIS solution dropwise to the free acid of the capped analog, stirring, and concentrating to obtain the capped composition.

A triethylamine salt of the capped analog is prepared by using an m7GDP imidazole salt and 5'-phosphorylated dinucleotide, followed by converting the triethylamine salt into a free acid of the capping analog through a cation exchange resin. A TRIS solution is added dropwise to the free acid of the capping analog for compounding; and stirring is continued. The amino groups of TRIS is paired with the negatively charged phosphate groups on nucleotides through positive and negative charge interactions in the solution. Concentrating the mixture affords the capped composition of the present disclosure. This process is simple. By adding the compounding step using a TRIS solution to the existing preparation process of the capping analog, the obtained final products have a higher capping efficiency and better intracellular protein expression and transcription and translation effect.

Preferably, the concentration of the TRIS solution is 0.3-0.7 mol/L.

By adopting the technical solution described herein, when the concentration of the TRIS salt solution used is more than 0.7 mol/L, a large amount of salt is left in the final product of the capped composition, thereby inhibiting biological enzymatic activities and reducing the yield of *in vitro* transcription and the capping efficiency of mRNA. When the concentration of the TRIS salt solution used is less than 0.3 mol/L, the capped composition may not form due to the low concentration of the TRIS salt solution.

Preferably, the TRIS solution is added to the free acid of the capped analog until a pH value of 3-7 is obtained for the TRIS salt solution.

By adopting the technical solution described herein, when the TRIS solution is added dropwise, the titration can be stopped when the pH is 3-7 for the solution comprising the capped composition, thereby obtaining the stable capped composition. If the pH is more than 7 or lower than 3, some chemical bonds on the phosphorylated nucleoside may decompose, and may result in unstable performance of the capped composition thus obtained and may affect the activity of the capping enzyme during mRNA transcription.

In a third aspect, the present disclosure provides an *in vitro* transcription reaction system, adopting the following technical solutions:

Provided is an *in vitro* transcription reaction system, comprising an RNA polymerase, a nucleoside triphosphate, and the capped composition described herein.

By adopting the technical solution described herein, an *in vitro* transcription reaction system comprises the RNA polymerase, the nucleoside triphosphate, and the capped composition of the present disclosure. Both the yield and the capping efficiency of mRNA are significantly improved after the transcription via the *in vitro* transcription system disclosed herein.

Preferably, the nucleoside triphosphate may be a natural nucleoside triphosphate, a modified nucleoside triphosphate, or an unnatural nucleoside triphosphate.

Preferably, the RNA polymerase is a phage-derived RNA polymerase.

More preferably, the RNA polymerase is T7, SP6, or T3.

Still more preferably, the RNA polymerase is T7.

In summary, the present disclosure displays at least the following technical effects:
1. The present disclosure describes compounding a capped analog with TRIS to prepare a salt solution, thereby obtaining a capped composition. When some specific mRNA sequences are transcribed, TRIS has better biocompatibility with mRNA, thereby increasing the yields of the mRNA transcription and the capping efficiency of mRNA.
2. The present disclosure discloses the concentration range of the added TRIS solution. If the concentration of the added TRIS solution exceeds the upper limit, a large amount of salt may be left in the final products of the capped composition, thereby inhibiting the biological enzymatic activity and reducing capping efficiency. If the concentration of the added TRIS solution is below the lower limit, the capped composition may not form due to the low concentration of the TRIS salt solution.
3. Experiments show that by compounding a capping analog with TRIS, the capping efficiency is significantly improved. The yield of mRNA synthesized by *in vitro* transcription is increased by 45% or more when compared to a sodium salt capped composition or an ammonium salt capped composition.
4. Experiments show that by compounding a capping analog with TRIS, the intracellular protein expression is increased 1.25 times or more when compared to a sodium salt capped composition or an ammonium salt capped composition.

### BRIEF DESCRIPTION OF THE DRAWINGS

FIG. 1 is a diagram showing a reaction scheme for the formula having 2-3 TRIS in Example 1.
FIG. 2 is a diagram showing a reaction scheme for the formula having 4-5 TRIS in Example 2.
FIG. 3 is diagram showing a reaction scheme for the formula having 6-7 TRIS in Example 3.
FIG. 4 is a diagram showing a reaction scheme for the formula having 2-3 sodium ions in Comparative Example 1.
FIG. 5 is a diagram showing a reaction scheme for the formula having 4-5sodium ions in Comparative Example 2.
FIG. 6 is a diagram showing a reaction scheme for the formula having 6-7 sodium ions in Comparative Example 3.
FIG. 7 is a diagram showing a reaction scheme for the formula having 4-5 ammonium cation in Comparative Example 4.
FIG. 8 is a diagram showing the translation effects of an intracellular mRNA protein when using Example 1, Comparative Example 1, and Comparative Example 4.

### EXAMPLES

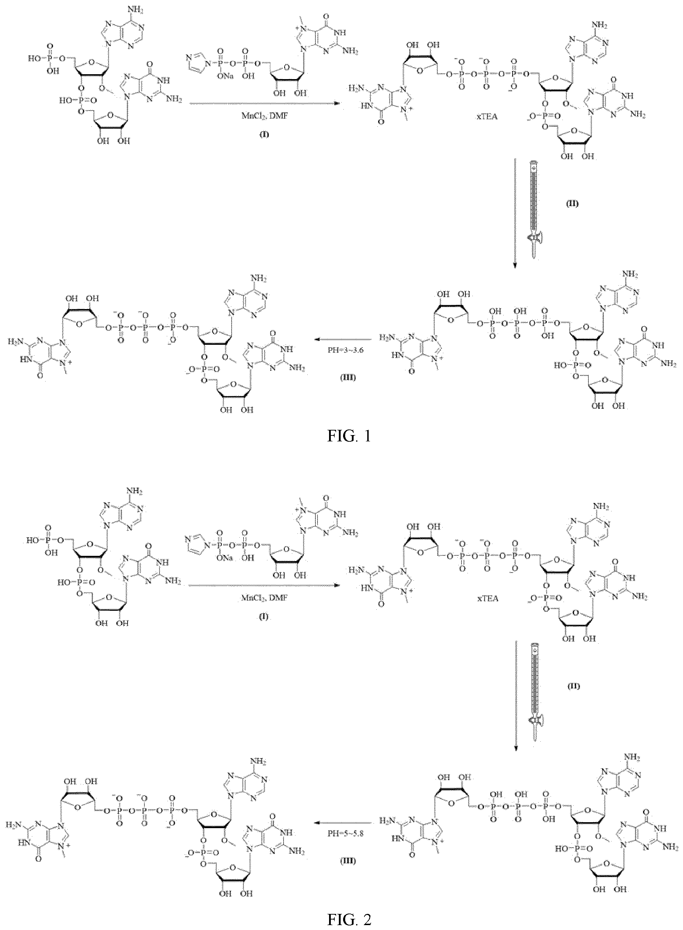

The present disclosure is described in further detail with reference to FIGs. 1-7 and Examples. All the starting materials are commercially available when used in the Examples and the Comparative Examples disclosed herein.

### Example 1:

Referring to FIG. 1, a capped composition was prepared by the following method:
I: Compound m7GDP imidazole salt (2 mmol) was dissolved in a DMF solution comprising MnCl₂ (2 mmol), and the solution was added to a DMF solution of 5'-phosphorylated dinucleotide (1 mmol; triethylamine salt). The mixture was stirred at room temperature. After 24 h, the reaction was quenched with 10 mL of a 0.25 M EDTA solution. The mixture was loaded onto a DEAESephadex column (3 × 50 cm). The product was eluted with a linear gradient of 0-1.0 M TEAB eluent. The eluted product fractions with an HPLC purity > 97% were collected and concentrated to obtain a 120 mM capped analog as a triethylamine salt (c).
II: The capped analog triethylamine salt was converted into a free acid form of the capped analog at the same concentration by a cation exchange resin.
III: TRIS base (0.2 M) was added dropwise to the free acid form of the capped analog until pH = 3-3.6. The mixture was concentrated, and the volume was adjustment to afford the final product, which is a system of a capped analog with 2-3 TRIS counterions.

### Example 2:

Referring to FIG. 2, a capped composition was prepared by the following method:
I: Compound m7GDP imidazole salt (2 mmol) was dissolved in a DMF solution comprising MnCl₂ (2 mmol), and the solution was added to a DMF solution of 5'-phosphorylated dinucleotide (1 mmol; triethylamine salt). The mixture was stirred at room temperature. After 24 h, the reaction was quenched with 10 mL of a 0.25 M EDTA solution. The mixture was loaded onto a DEAESephadex column (3 × 50 cm). The product was eluted with a linear gradient of 0-1.0 M TEAB eluent. The eluted product fractions with an HPLC purity > 97% were collected and concentrated to obtain a 120 mM capped analog as a triethylamine salt.
II: The capped analog triethylamine salt was converted into a free acid form of the capped analog at the same concentration by a cation exchange resin.
**III:** TRIS base (0.2 M) was added dropwise to the free acid form of the capped analog until pH = 5-5.8. The mixture was concentrated, and the volume was adjustment to afford the final product, which is a system of a capped analog with 4-5 TRIS counterions.

### Example 3:

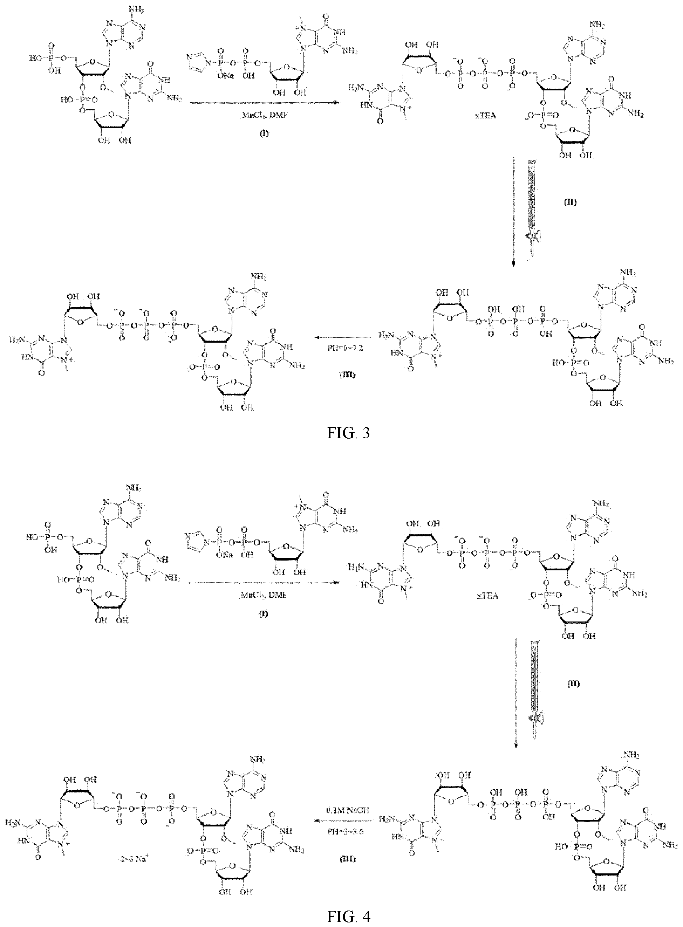

Referring to FIG. 3, a capped composition was prepared by the following method:
I: Compound m7GDP imidazole salt (2 mmol) was dissolved in a DMF solution comprising MnCl₂ (2 mmol), and the solution was added to a DMF solution of 5'-phosphorylated dinucleotide (1 mmol; triethylamine salt). The mixture was stirred at room temperature. After 24 h, the reaction was quenched with 10 mL of a 0.25 M EDTA solution. The mixture was loaded onto a DEAESephadex column (3 × 50 cm). The product was eluted with a linear gradient of 0-1.0 M TEAB eluent. The eluted product fractions with an HPLC purity > 97% were collected and concentrated to obtain a 120 mM capped analog as a triethylamine salt.
II: The capped analog triethylamine salt (c) was converted into a free acid form of the capped analog at the same concentration by a cation exchange resin.
**III:** TRIS base (0.2 M) was added dropwise to the free acid form of the capped analog until pH = 6-7.2. The mixture was concentrated, and the volume was adjustment to afford the final product, which is a system of a capped analog with 6-7 TRIS counterions.

### Comparative Example 1:

Referring to FIG. 4, a capped composition having sodium salt was prepared by the following method:
I: Compound m7GDP imidazole salt (2 mmol) was dissolved in a DMF solution comprising MnCl₂ (2 mmol), and the solution was added to a DMF solution of 5'-phosphorylated dinucleotide (1 mmol; triethylamine salt). The mixture was stirred at room temperature. After 36 h, the reaction was quenched with 10 mL of a 0.25 M EDTA solution. The mixture was loaded onto a DEAESephadex column (3 × 50 cm). The product was eluted with a linear gradient of 0-1.0 M TEAB eluent. The eluted product fractions with an HPLC purity > 97% were collected, concentrated, and desalted to obtain a capped analog as a triethylamine salt.
II: The capped analog triethylamine salt was converted into a free acid form of the capped analog at the same concentration by a cation exchange resin.
**III:** NaOH solution (0.1 M) was added dropwise to the free acid form solution until pH = 3-3.6. The mixture was concentrated, and the volume was adjusted to afford a 100 mM final product of a capped analog with 2-3 sodium counterions.

### Comparative Example 2:

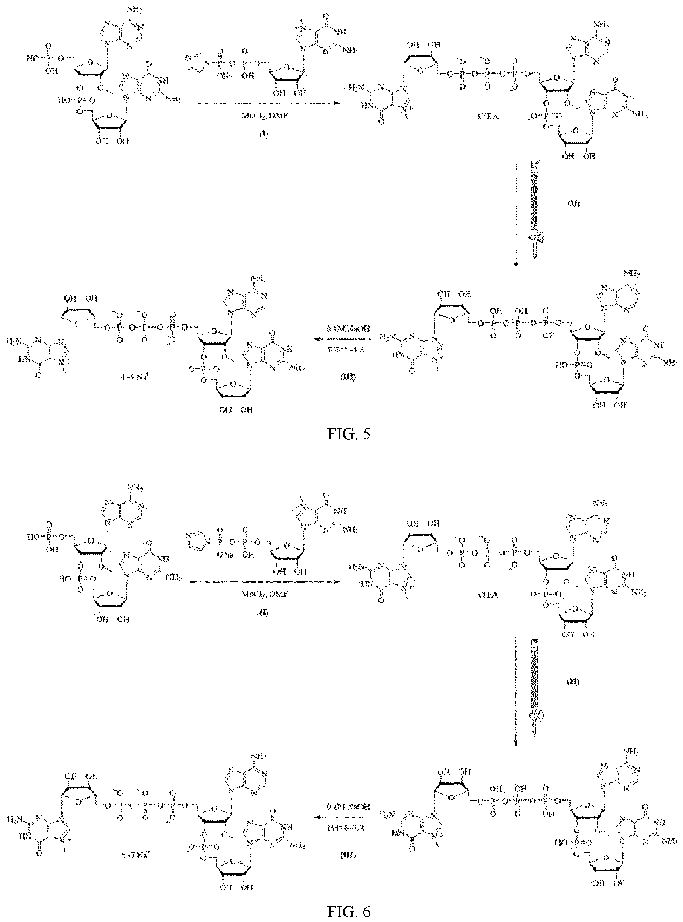

Referring to FIG. 5, a capped composition sodium salt was prepared by the following method:
I: Compound m7GDP imidazole salt (2 mmol) was dissolved in a DMF solution comprising MnCl₂ (2 mmol), and the solution was added to a DMF solution of 5'-phosphorylated dinucleotide (1 mmol; triethylamine salt). The mixture was stirred at room temperature. After 36 h, the reaction was quenched with 10 mL of a 0.25 M EDTA solution. The mixture was loaded onto a DEAESephadex column (3 × 50 cm). The product was eluted with a linear gradient of 0-1.0 M TEAB eluent. The eluted product fractions with an HPLC purity > 97% were collected, concentrated, and desalted to obtain a capped analog as a triethylamine salt.
II: The capped analog triethylamine salt was converted into a free acid form of the capped analog at the same concentration by a cation exchange resin.
III: NaOH solution (0.1 M) was added dropwise to the free acid form solution until pH = 5-5.8. The mixture was concentrated, and the volume was adjusted to afford a 100 mM final product of a capped analog with 4-5 sodium counterions.

### Comparative Example 3:

Referring to FIG. 6, a capped composition sodium salt was prepared by the following method:
I: Compound m7GDP imidazole salt (2 mmol) was dissolved in a DMF solution comprising MnCl₂ (2 mmol), and the solution was added to a DMF solution of 5'-phosphorylated dinucleotide (1 mmol; triethylamine salt). The mixture was stirred at room temperature. After 36 h, the reaction was quenched with 10 mL of a 0.25 M EDTA solution. The mixture was loaded onto a DEAESephadex column (3 × 50 cm). The product was eluted with a linear gradient of 0-1.0 M TEAB eluent. The eluted product fractions with an HPLC purity > 97% were collected, concentrated, and desalted to obtain a capped analog as a triethylamine salt.
II: The capped analog triethylamine salt was converted into a free acid form of the capped analog at the same concentration by a cation exchange resin.
III: NaOH solution (0.1 M) was added dropwise to the free acid form solution until pH = 6-7.2. The mixture was concentrated, and the volume was adjusted to afford a 100 mM final product of a capped analog with 6-7 sodium counterions.

### Comparative Example 4:

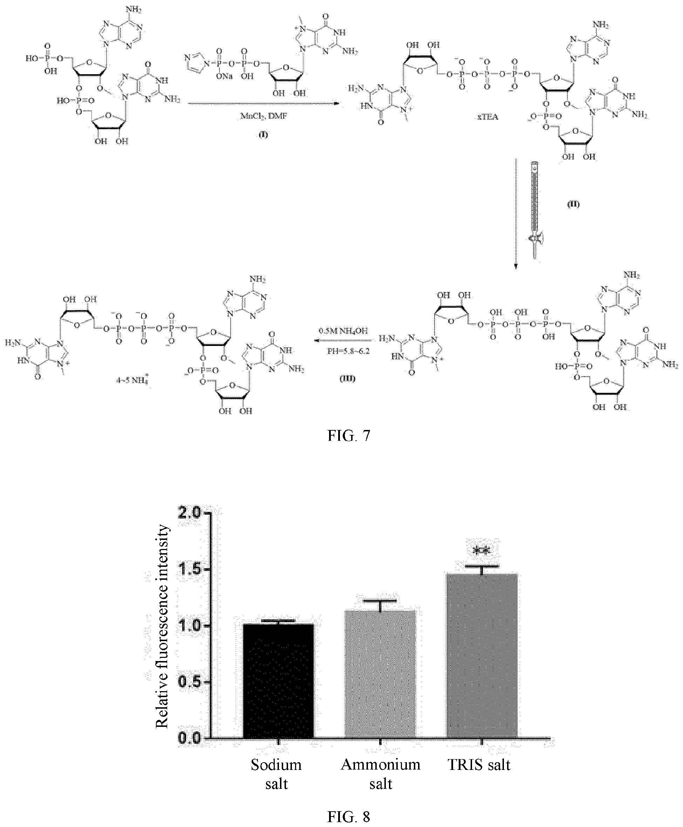

Referring to FIG. 7, a capped composition ammonium salt was prepared by the following method:
I: Compound m7GDP imidazole salt (2 mmol) was dissolved in a DMF solution comprising MnCl₂ (2 mmol), and the solution was added to a DMF solution of 5'-phosphorylated dinucleotide (1 mmol; triethylamine salt). The mixture was stirred at room temperature. After 36 h, the reaction was quenched with 10 mL of a 0.25 M EDTA solution. The mixture was loaded onto a DEAESephadex column (3 × 50 cm). The product was eluted with a linear gradient of 0-1.0 M TEAB eluent. The eluted product fractions with an HPLC purity > 97% were collected, concentrated, and desalted to obtain a capped analog as a triethylamine salt.
II: The capped analog triethylamine salt was converted into a free acid form of the capped analog at the same concentration by a cation exchange resin.
III: Aqueous ammonia solution (0.5 M) was added dropwise to the free acid form solution until pH = 5.8-6.2. The mixture was concentrated, and the volume was adjusted to afford a 100 mM final product of a capped analog with 4-5 ammonium cation counterions.

### Comparative Example 5:

A capped composition was prepared by the following method:
I: Compound m7GDP imidazole salt (2 mmol) was dissolved in a DMF solution comprising MnCl₂ (2 mmol), and the solution was added to a DMF solution of 5'-phosphorylated dinucleotide (1 mmol; triethylamine salt). The mixture was stirred at room temperature. After 24 h, the reaction was quenched with 10 mL of a 0.25 M EDTA solution. The mixture was loaded onto a DEAESephadex column (3 × 50 cm). The product was eluted with a linear gradient of 0-1.0 M TEAB eluent. The eluted product fractions with an HPLC purity > 97% were collected and concentrated to obtain a 120 mM capped analog as a triethylamine salt.
II: The capped analog triethylamine salt was converted into a free acid form of the capped analog at the same concentration by a cation exchange resin.
III: TRIS base (0.2 M) was added dropwise to the free acid form solution until pH = 2.3-2.8. The mixture was concentrated, and the volume was adjusted to afford the final product, which is a system of a capped analog with 1-2 TRIS counterions.

### Comparative Example 6:

A capped composition was prepared by the following method:
I: Compound m7GDP imidazole salt (2 mmol) was dissolved in a DMF solution comprising MnCl₂ (2 mmol), and the solution was added to a DMF solution of 5'-phosphorylated dinucleotide (1 mmol; triethylamine salt). The mixture was stirred at room temperature. After 24 h, the reaction was quenched with 10 mL of a 0.25 M EDTA solution. The mixture was loaded onto a DEAESephadex column (3 × 50 cm). The product was eluted with a linear gradient of 0-1.0 M TEAB eluent. The eluted product fractions with an HPLC purity > 97% were collected and concentrated to obtain a 120 mM capped analog as a triethylamine salt.
II: The capped analog triethylamine salt was converted into a free acid form of the capped analog at the same concentration by a cation exchange resin.
III: TRIS base (0.2 M) was added dropwise to the free acid form solution until pH = 7.5-8.2. The mixture was concentrated, and the volume was adjusted to afford the final product, which is a system of a capped analog with 7-8 TRIS counterions.

Biological indexes such as IVT yield, capping efficiency, intracellular target protein expression, and the like were compared for three capped analogs, i.e., the capped composition sodium salt, the capped composition ammonium salt, and the capped composition of the present disclosure.

*In vitro* synthesis of mRNA using capped compositions

### (1) Preparation of DNA template

Typical templates are linearized plasmids, PCR amplification products, or synthetic DNA fragments. To prepare a linearized plasmid template, a target fragment is usually inserted into the multiple cloning site of a plasmid by means of digestion, recombination, or the like, and the plasmid generally has a promoter site of the corresponding RNA polymerase, such as T7, SP6, or T3. The plasmid was then amplified in a "biological factory" such as *Escherichia coli,* and the like. After extraction and purification, the modified plasmid is cleaved and purified by a restriction endonuclease. The purified product is used as a DNA template for *in vitro* transcription of mRNA. To prepare a PCR amplification product, a DNA polymerase, a specific primer, and a related buffer are usually used as a system to perform amplification on a target DNA fragment. The amplification product usually includes a promoter site of the corresponding RNA polymerase, such as T7, SP6, T3, or the like. In this example, a T7 RNA polymerase is used. After purification, the amplification product can be used as a DNA template for *in vitro* transcription of mRNA.

### (2) In vitro transcription system

**Table 1. Composition of in vitro transcription system of mRNA**

| Systems | Amount |
|---|---|
| T7RNA polymerase | 50 U |
| 10× buffer | 2 µL |
| 100 mM ATP | 1 µL |
| 100 mM GTP | 1 µL |
| 100 mM CTP | 1 µL |
| 100 mM N1-Me-pUTP | 1 µL |
| 100 mM capped composition | 1 µL |
| Inorganic pyrophosphatase | 0.05 U |
| Nuclease inhibitor | 20 U |
| Sterile enzyme-free water | added till 20 µL |
| DNA template | 1 µg |

In the table, the capped compositions are: the capped composition sodium salt, the capped composition ammonium salt, or the capped composition TRIS salt.

In the experimental process, firstly, sterile enzyme-free water was added to the system, and then 10× buffer, ATP, GTP, CTP, N1-Me-pUTP, and the capped composition were sequentially added. The mixture was gently centrifuged after mixing, followed by the addition of the nuclease inhibitor, the inorganic pyrophosphatase, the T7RNA polymerase, and the DNA template. The mixture was centrifuged after mixing and incubated at 37 °C. After 2 h, DNase I (1 U) was added, and the mixture was incubated at 37 °C for another 30 min to remove the DNA template. Then the RNA was purified.

### (3) Yields of mRNA

The purified mRNA was dissolved in sterile enzyme-free water, and was quantitatively detected by using a UV spectrophotometer NanodropOne. The results are shown in Table 2.

**Table 2. Yields of mRNA transcribed in vitro using different initiating capped compositions**

| Samples | Yields (µg) |
|---|---|
| Example 1 | 72 |
| Example 2 | 74 |
| Example 3 | 73 |
| Comparative Example 1 | 47 |
| Comparative Example 2 | 48 |
| Comparative Example 3 | 47 |
| Comparative Example 4 | 49 |
| Comparative Example 5 | 56 |
| Comparative Example 6 | 60 |

The detection results in Table 2 in view of Examples 1-3 and Comparative Examples 1-4 show that the yield of mRNA is remarkably increased to 72-74 µg when the capped composition of the present disclosure is used for synthesizing mRNA by *in vitro* transcription, while the yields of mRNA are remarkably reduced to 47-49 µg when a capped composition sodium salt or a capped composition ammonium salt is used. The experimental results show that the yield of synthesized mRNA is increased 46.9-57.4% when the composition of the present disclosure is used for *in vitro* transcription, dramatically exceeding the expectation for the experiments.

The detection results in Table 2 in view of Examples 1-3 and Comparative Examples 5-6 show that when the pH of the capped composition is outside the range of pH 3-7, that is, when the molar ratio of the capped analog to TRIS is outside the range of 1:(3-7), the yields of mRNA are greatly reduced. The reason may be that some chemical bonds on the phosphorylated nucleoside may decompose, and may result in unstable performance of the capped composition thus obtained and may affect the activity of the capping enzyme during mRNA transcription.

### (4) Capping efficiency comparison

Liquid chromatography-mass spectrometry (LC-MS) was used to detect IVT capping efficiency of mRNA with different initiating capped analogs. Firstly, a labeled (usually biotinlabeled) DNA probe was designed to pair with the starting base of the transcription product mRNA. Streptavidin-labeled magnetic beads were washed and then incubated with the synthesized DNA probe, mRNA, and 10× RNase H reaction buffer with slow mixing at room temperature for 30 min. RNase H (5 U/µL, 20 µL) was then added, and the mixture was incubated at 37 °C for 3 h, with mixing every half an hour. After the incubation was completed, the magnetic beads were washed. The washed magnetic beads were added to 100 µL of 75% methanol heated to 80 °C. The mixture was heated to 80 °C on a heating plate, held for 3 min, and then placed on a magnetic rack. The supernatant was pipetted. Dried at room temperature for 45 min to 10 µL by using an evaporation centrifuge. The sample was then resuspended in 50 µL of 100 µM EDTA/1% MeOH for LC-MS analysis to determine the capping of RNA in the transcription reaction. Due to the significant difference in molecular weight between capped and uncapped bases, the capping efficiencies of mRNA transcription initiated with different capped analogs can be determined by using the difference in molecular weight. The specific results are shown in Table 3 and mass spectra for the same batch of experiments.

**Table 3. Capping efficiencies of mRNA transcribed in vitro using different initiating capped compositions**

| Capped Compositions | Capping Efficiency (%) |
|---|---|
| Example 1 | 98.4 |
| Example 2 | 99.7 |
| Example 3 | 99.2 |
| Comparative Example 1 | 91.55 |
| Comparative Example 2 | 92.67 |
| Comparative Example 3 | 90.89 |
| Comparative Example 4 | 93.84 |
| Comparative Example 5 | 92.86 |
| Comparative Example 6 | 92.79 |

The detection results in Table 3 in view of Examples 1-3 and Comparative Examples 1-4 show that the capping efficiency of mRNA can reach 99.7% when the capped composition of the present disclosure is used for synthesizing mRNA by *in vitro* transcription, while the capping efficiency is drastically reduced to lower than 95% when capped composition sodium salt or the capped composition ammonium salt is used. The experimental results show that the capping efficiency of mRNA is significantly increased when the composition of the present disclosure is used for *in vitro* transcription, dramatically exceeding the expectation for the experiments.

The detection results in Table 3 in view of Examples 1-3 and Comparative Examples 5-6 show that when the pH of the capped composition is outside the range of pH 3-7, that is, when the molar ratio of the capping to TRIS is outside the range of 1:(3-7), the capping efficiency of mRNA is greatly reduced. the yields of mRNA are greatly reduced. The reason may be that some chemical bonds on the phosphorylated nucleoside may decompose, and may result in unstable performance of the capped composition thus obtained and may affect the activity of the capping enzyme during mRNA transcription, thereby affecting the improvement of mRNA capping efficiency.

### (5) Protein expression in cells

In this example, the eGFP coding sequence was used as a DNA template, and the capped compositions of Comparative Example 1, Comparative Example 4, and Example 1 were selected as the initiating capped analogs for *in vitro* transcription. The specific capping is performed using the sodium salt, the ammonium salt, or the TRIS salt of ^{m 7}GpppA_{2'O me} pG. Each of the different mRNA products thus obtained was subsequently transfected into 293T cell. Specifically, the 293T cells were plated into a plate (24-well plate) at 0.5-1 × 10⁵ cells. Transfection experiments with cells within 50 passages are recommended. The cells were required to be passaged again 24 h before the transfection. The addition of antibiotics to the culture medium did not affect the transfection effect. During the transfection, in general, the cell density was preferably 60-80%; 2 µg of mRNA was transfected into each well, and the Lipofectamine Messenger MAX Transfection Reagent (Invitrogen) was selected as the transfection reagent and used according to the manufacturer instructions. The transfected cells were placed in an incubator at 37 °C with CO₂. After transfection for 4-6 h, the culture medium was replaced with a fresh complete culture medium. After incubation in the incubator at 37 °C with CO₂ for 24 h, the fluorescence intensity of GFPs from the cells was observed via a fluorescence microscope.

The intracellular mRNA protein translation effects in FIG. 8 for Example 1, Comparative Example 1, and Comparative Example 4 indicate that the mRNA protein translation effect obtained by *in vitro* transcription using the capped composition of the present disclosure is 1.5 times or more when compared with using the capped composition sodium salt, and 1.25 times or more when compare with using the capped composition ammonium salt. Accordingly, the expression effect and translation effect of mRNA in cells are better when the capped composition compounding with TRIS is used for *in vitro* transcription.

## Claims

1. A capped composition, wherein the composition is substantially composed of a salt solution formed by compounding of a capping analog with TRIS according to a molar ratio of 1:(3-7).

2. The capped composition according to claim 1, wherein the capping analog has a formula of 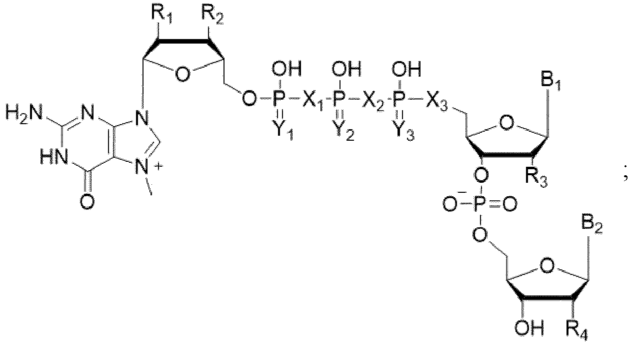
the TRIS has a formula of 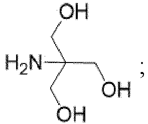
wherein, each of B₁ and B₂ is independently a natural or modified base;
R₁ is H, OH, alkyl, O-alkyl, or halogen;
R₂ is H, OH, alkyl, O-alkyl, or halogen;
R₃ is hydrogen, OH, substituted or unsubstituted O-alkyl, substituted or unsubstituted S-alkyl, substituted or unsubstituted NH-alkyl, substituted or unsubstituted N-dihydrocarbyl, substituted or unsubstituted O-aryl, substituted or unsubstituted S-aryl, substituted or unsubstituted NH-aryl, substituted or unsubstituted O-arylalkyl, substituted or unsubstituted S-arylalkyl, or substituted or unsubstituted NH-arylalkyl;
R₄ is H, OH, substituted or unsubstituted O-alkyl, substituted or unsubstituted S-alkyl, substituted or unsubstituted NH-alkyl, substituted or unsubstituted N-dihydrocarbyl, substituted or unsubstituted O-aryl, substituted or unsubstituted S-aryl, substituted or unsubstituted NH-aryl, substituted or unsubstituted O-arylalkyl, substituted or unsubstituted S-arylalkyl, or substituted or unsubstituted NH-arylalkyl;
each of X₁, X₂, and X₃ is independently O, CH₂, or NH; and
each of Y₁, Y₂, and Y₃ is independently O, S, Se, or BH₃.

3. The capped composition according to claim 2, wherein the capping efficiency when using the capped composition is 98% or more.

4. The capped composition according to claim 2, wherein the capped composition increases the yield of mRNA by 45% or more in *in vitro* transcription, as compared to a sodium salt capped composition or an ammonium salt capped composition.

5. The capped composition according to claim 2, wherein the capped composition increases the intracellular protein translation efficiency of mRNA by 1.25 times or more after *in vitro* transcription, as compared to a sodium salt capped composition or an ammonium salt capped composition.

6. A method of making the capped composition according to any one of claims 1-5, comprising the following steps:
dissolving an m7GDP imidazole salt in a DMF solution comprising MnCl₂, adding the DMF solution of m7GDP imidazole salt to a DMF solution of 5'-phosphorylated dinucleotide, stirring the mixture at room temperature, and concentrating to obtain a triethylamine salt of the capped analog;
converting the triethylamine salt of the capped analog to a free acid of the capped analog by a cation exchange resin; and
adding a TRIS solution dropwise to the free acid of the capped analog, stirring, and
concentrating to obtain the capped composition.

7. The method of making the capped composition according to claim 6, wherein the concentration of the TRIS solution is 0.3-0.7 mol/L.

8. The method of making the capped composition according to claim 6, wherein the TRIS solution is added to the free acid of the capped analog until a pH value is 3-7 for the salt solution.

9. An *in vitro* transcription reaction system, comprising an RNA polymerase, a nucleoside triphosphate, and the capped composition according to any one of claims 1-5.

10. The *in vitro* transcription reaction system according to claim 9, wherein the nucleoside triphosphate is a natural nucleoside triphosphate, a modified nucleoside triphosphate, or an unnatural nucleoside triphosphate; and wherein the RNA polymerase is T7, SP6, or T3.
